# EUROPEAN PATENT SPECIFICATION

(11) **EP 4 306 033 B1**
(45) Date of publication and mention of the grant of the patent: **12.03.2025**
(21) Application number: 22184709.8
(22) Date of filing: 13.07.2022
(51) Int. Cl.: A61B 1/005

(54) **ENDOSCOPE WITH CONE BRAKE**
ENDOSKOP MIT KONUSBREMSE
ENDOSCOPE DOTÉ D'UN FREIN CONIQUE

(43) Date of publication of application: 17.01.2024
(73) Proprietor: Ambu A/S, 2750 Ballerup (DK)
(72) Inventor: CHRISTENSEN, Martin Johst, 2100 Copenhagen Ø (DK); NIELSEN, Martin Refslund, 3460 Birkerød (DK)
(74) Representative: Winter, Brandl - Partnerschaft mbB

(56) References cited:
- US-A- 5 329 887
- US-A- 5 507 717
- US-A1- 2015 359 415
- US-A1- 2021 338 049
- US-A1- 2022 151 463

## Description

The present disclosure relates to an endoscope comprising a proximal endoscope handle or interface having a handle or interface housing, an insertion cord extending from the endoscope handle or interface and configured to be inserted into a patient's body cavity, the insertion cord comprising an insertion tube, a bending section and a distal tip unit, and a steering mechanism configured to swivel the distal tip unit by bending the bending section at least in a first bending plane, the steering mechanism comprising a manually operable first steering input element being provided for receiving a rotational steering input by a user and being rotatably supported at the handle or interface housing, and a first braking mechanism for braking the first steering input element with respect to the handle or interface housing.

### Related Art

Steerable endoscopes often have a proximal endoscope handle and a distal insertion cord including a bending section and a distal tip unit. In order to deflect the distal tip unit, the bending section can be bent or manipulated by pulling one or more pulling/ steering wires, which extend into the insertion cord of the endoscope and which have distal portions attached to the bending section.

Typically, in an endoscope, which allows four-way-bending, four pulling wires may be provided, which are circumferentially offset with respect to each other by 90°, and pulling either one of the pulling wires/ steering wires will result in a bending motion in a corresponding direction. Thus, two degrees of freedom (two bending planes) are provided with one degree of freedom comprising an up/ down movement of the bending section effected by pulling one of two diametrically opposing pulling wires, and another degree of freedom comprising a left/ right movement of the bending section effected by pulling one of two other diametrically opposing pulling wires.

Such endoscopes are generally provided with two steering input wheels (also called manually operable wheels/ wheel manipulators) at the endoscope handle, with one of the steering input wheels being provided to control an up/down bending and the other one of the steering input wheels being provided to control a left/ right bending. The steering input wheels or wheel manipulators are coaxially arranged (stacked next to each other) adjacent to a handle housing. Each steering input wheel is connected to a control wheel, such as a sprocket or wire drum, which is connected to two of the steering wires, e.g. via a chain engaging the sprocket or by winding the wire around the drum. Thus, the steering input wheel is rotated to rotate the control wheel, which results in a pulling of the one (first) wire connected thereto and a loosening of the other (second) wire connected thereto.

Similarly, endoscopes which allow two-way-bending generally comprise only two pulling wires circumferentially offset by 180° and connected to only one control wheel which is operable by only one steering input wheel.

Further, such endoscopes may have braking mechanisms including braking input elements or brake activators such as a rotatable brake knob. In particular, one braking mechanism exists for braking each wheel manipulator. When the braking mechanisms are applied, the position of the wheel manipulator will be fixed in the current orientation. This allows holding the distal tip in a desired orientation without holding the steering input wheels manually. In case of a four-way-bending endoscope, the braking input element or brake activator for braking the up-down control wheel is typically a lever and for braking the left-right control wheel is a rotatable knob.

Such braking mechanisms typically include a stack of braking discs and a large number of additional parts. For example, for braking each wheel manipulator, three sets of discs respectively comprising one static disc and one rotating disc may be provided, the static discs being non-rotatable relative to a handle housing of the endoscope handle and the rotating discs being rotatable together with the wheel manipulator. Further parts include a disc hold for holding the stack of discs, a spring for biasing or compressing the discs, a spring hold for holding the spring, and a ramp for transforming a rotary input at the braking input element into an axial translation of the spring.

In operation, the brake knob is rotated and comes into contact with the ramp, which translates to the brake disc stack and compresses the spring. A force of said spring is transmitted through the stack of discs and in every interface between the discs a friction force is applied which works against the rotational movement of the corresponding wheel manipulator.

Such braking mechanisms function well in terms of providing an adequate braking force. However, they comprise a large number of small parts which are weak and difficult to handle because of their low rigidity.

An endoscope with a similar mechanism including one or more braking discs is e.g. known from US 2001/0034472 A1. Prior art documents US 2021/338049, US 2015/359415, US 5 507 717, US 5 329 887 and US 2022/151463 all disclose endoscopes according to the preamble of claim 1.

### Brief description of the disclosure

In view of the above-described problems it is an object of the present disclosure to provide an endoscope which shall reduce or avoid the disadvantages of the related art. In particular, it is an object of the disclosure to provide an endoscope with a steering mechanism for steering a bending section of an insertion cord and including a braking mechanism which is simple and cost-effective to manufacture and to assemble.

This object is solved by an endoscope in accordance with claim 1 and by a system in accordance with claim 15. Advantageous aspects of the present disclosure are claimed in the dependent claims and/or are explained below.

In detail, the present disclosure relates to an endoscope comprising: a proximal endoscope handle or interface having a handle or interface housing; an insertion cord extending from the endoscope handle or interface and configured to be inserted into a patient's body cavity, the insertion cord comprising an insertion tube, a bending section and a distal tip unit; and a steering mechanism configured to swivel the distal tip unit by bending the bending section at least in a first bending plane, the steering mechanism comprising a manually operable first steering input element being provided for receiving a rotational steering input by a user and being rotatably supported at the handle or interface housing; and a first braking mechanism for braking the first steering input element with respect to the handle or interface housing, the first braking mechanism comprising a manually operable first braking input element being provided for receiving a braking input by a user, and a first braking cone element or portion which forms an essentially cone-shaped first braking surface and is non-rotatably connected to the handle or interface housing, and a second braking cone element or portion which forms an essentially cone-shaped second braking surface and is non-rotatably connected to the first steering input element or portion, with the first braking cone element or portion and/ or the second braking cone element or portion being axially translatable via the first braking input element to press the first braking surface against the second braking surface.

The first steering input element may be fixed to a first input shaft forming a control wheel supporting and controlling steering wires. The first control shaft may be supported to be rotatable around a central axis relative to the handle or interface housing. Expressions such as "axially", "radially" and "circumferentially" relate to directions in relation to a central axis of the first input shaft. Expressions such as "axially outwards" or "axially inwards" correspondingly refer to a direction outwards or inwards with respect to the handle or interface housing viewed in a direction along the central axis of the first input shaft, i.e. away from or towards the housing.

Expressed in other words, the endoscope according to the present disclosure has an endoscope handle or interface with a handle or interface housing and an insertion portion with a steerable bending section which is steerable via a steering mechanism comprising a steering input wheel and a braking mechanism. The braking mechanism comprises a pair of braking surfaces formed of a first braking surface and a second braking surface (the first and second braking surfaces), at least one of which is axially shiftable or translatable to come into or out of braking contact with the other one thereof. The first and second braking surfaces are essentially cone shaped. Their cone shapes may preferably have walls extending in a straight diagonal line, i.e. a true cone geometry. Alternatively, the cone shapes may have rounded contours, e.g. a convex contour such as a barrel section, a sphere section or an ellipsoid section or a concave contour such as a trumpet section. Further variants are discussed in more detail below.

This is advantageous since the friction generating surfaces on the braking surfaces, i.e. first and second braking surfaces, are arranged relatively far radially outwards compared to simple flat discs, and the area of the friction generating surfaces may be larger without increasing the diameter. A braking torque generated via braking surfaces is calculated based on a braking force pressing the braking surfaces against each other and a lever determined by a distance between a location where said braking force acts on the braking surfaces and the central axis. Thus, since the braking surfaces are arranged relatively far radially outwards the lever for the braking force generated between said first and second braking surfaces is large and the resulting braking torque is large. This allows to reduce the number of friction generating surfaces necessary for generating an appropriate braking force. Accordingly, a number of braking surfaces or of braking cone elements may be reduced, the braking mechanism in particular compromising only three to five parts in total. Thus, assembly is simplified and costs are reduced. In addition, the cone shaped parts are more robust than thin friction discs and thus damage to the braking mechanism in particular during assembly is reduced. Due to the smaller number of plastic parts required, the present solution is moreover better for the environment.

When the first braking surface is pushed against the second braking surface, the first braking mechanism is closed and rotation of the first steering input element relative to the handle or interface housing is restrained. Preferably, the first and second braking surfaces are the only area of contact between the first and second braking cone elements or portions. In particular, an axial gap may be formed between their opposing front sides. When the first braking mechanism is open or not closed, the first steering mechanism is, in particular freely, rotatable. In this state, a gap is preferably formed between the first and second braking surfaces. Preferable, the first and second braking surfaces may be brought directly into braking contact with each other. Alternatively, additional intermediate braking elements may be provided. One of the first and second braking surfaces is a radially inner cone surface and the other one is a radially outer cone surface. The first braking cone element or portion and the second braking cone element or portion may respectively be a portion of another member of the steering mechanism or may be separate members. In particular, the second braking cone element or portion may be formed integrally with the first steering input element.

The manually operable first braking input element is preferably formed as a rotatable knob. Alternatively, it may e.g. be a lever. The first steering input element may preferably be a rotatable manipulator wheel. The first steering input element and the first braking input element may be coaxial with respect to each other. The first input shaft may form or may be connected to a first control wheel (i.e. wire drum or a first sprocket) which transforms a rotation of the first input shaft into a translation of one or more steering wires which extend through the insertion section to the bending section to effect a bending of said bending section in the first bending plane. Above mentioned non-rotatable and axially translatable connections may preferably be provided via a non-circular cross-sectional shaft or rod, e.g. via an axially extending spline geometry between the corresponding members. Preferably, only one among the first and second braking cone elements or portions is axially translatable. Optionally, both may be axially translatable.

Preferably, the first braking input element forms a first cam structure and the first braking cone element or portion comprises a second cam structure which contacts the first cam structure such that a rotation of the first braking input element is transformed into an axial translation of the first braking cone element or portion. This is a particularly simple, construction space-efficient and cost-efficient mechanism. Preferably, the first cam structure may be a cam formed at a protrusion protruding axially from the first braking input element and the second cam structure may be a surface contacting said cam. In this case, the cam as the more fragile structure in a cam pairing comprising a cam and cam surface is formed at the larger and easier handleable member among the first braking input element and the first braking cone element or portion. Accordingly, this allows the use of more robust members which are less prone to damage during assembly.

In particular, the first cam structure and/ or the second cam structure may comprise a first ramp portion extending circumferentially and axially with respect to the first shaft.

The first ramp portion preferably has a continuous inclination. This enables a user to operate the first braking input element with an even, continuous amount of force. Alternatively, it may also have a rounded contour.

Further, it is preferable that the first cam structure and/ or the second cam structure form a first end portion (particularly a first flat end portion) at one side of the first ramp portion and structured differently than the first ramp portion, said first end portion defining an axial position of the first braking cone element or portion when the first braking mechanism is opened. In this case, the user receives a tactile feedback when the first braking mechanism is fully opened. Additionally or alternatively, it is preferable that the first cam structure and/ or the second cam structure form a second end portion (particularly a second flat end portion) at another side of the first ramp portion (i.e. opposite the first flat end portion), structured differently than the first ramp portion and defining an axial position of the first braking cone element or portion when the first braking mechanism is closed. This provides a tactile feedback to the user upon reaching such a closed state and further allows reproducible setting of a defined braking force. Also, such end portions provide a holding force e.g. hindering the cam from sliding back down the first ramp surface.

The first end portion or the second end portion may be formed by an axial front surface of the first braking cone element or portion. Alternatively, the first end portion and/ or the second end portion may be formed by bumps or recesses or the like. Additional flat or bumpy or recessed portions may be provided along the first ramp portion e.g. to provide feedback upon reaching a predefined intermediate braking force.

It is considered to be advantageous to provide a first biasing or spring element, which biases the first cam structure towards the second cam structure. Thus, contact between the first and second cam structures during operation of the first braking input element can be ensured. Further, it is preferable if the first biasing or spring element biases the first braking cone element or portion towards a closed position in which the first braking mechanism is closed. In this case, the braking force provided when the first braking mechanism is closed (i.e. a closed state) is defined by the first spring element. Thus, a predefined, reproducible braking force can be achieved in the closed state. In particular, the predefined braking force can be easily adjusted by exchanging the biasing or spring element without needing to amend other possibly standardized components. The first biasing or spring element is preferably a mechanical spiral spring. Other biasing or spring elements such as leaf springs, magnets or the like may alternatively be used.

The first biasing or spring element may be arranged between the first braking input element and the first braking cone element or portion. This is particularly advantageous, since it allows to form the second braking cone element or portion integrally with the first steering input element. Thus, no additional member is needed as the second braking cone element and costs as well as assembly time and assembly errors may be reduced.

Alternatively, the first biasing or spring element may be arranged between the second braking cone element or portion and the first steering input element. This is considered advantageous since the second braking cone element or portion and the first steering input element are non-rotatably connected to each other. Thus, scratching of axial ends of the first biasing or spring element at their axial supporting surfaces may be avoided. Accordingly, wear of the parts e.g. releasing plastic particles which may lead to contamination or the like can be reduced.

In particular, it is considered advantageous if the first biasing or spring element (alteratively called a flexing element or a compressive element) is supported radially inside at the first braking cone element at a side opposite to the first braking surface. Alternatively, the first biasing or spring element may be supported radially inside at the second braking cone element at a side opposite to the second braking surface. In the latter two cases, an axial position of the first biasing or spring element and the first or respectively second braking surface may overlap. In other words, the first biasing or spring element may be nested / at least partially accommodated inside the first or respectively second braking cone element. Due to this, a required axial construction space is small.

Advantageously, the first braking cone element or portion forms at least one circumferential slit and the first braking input element forms at least one first hook extending through the at least one circumferential slit to pull the first braking cone element or portion.

Expressed in other words, the first braking input element may form a protrusion penetrating the first braking cone element and at an end of said protrusion may form a radially extending nose portion for reaching behind an edge of the first braking cone element or portion in order to pull it towards the first braking input element. This is particularly advantageous, if the at least one first hook pulls the first braking cone element or portion away from the second braking cone element or portion. In this case, a risk of the first and second braking cone elements or portions sticking together due to a wedging effect or the like can be reduced. In this case, it is further preferable if an axial position or movement of the second braking cone element or portion in a direction towards the first braking input element is limited. This can be achieved e.g. by the second braking cone element or portion being fixedly connected to the first steering input element or by an axial stop being formed, preferably by the first steering input element, for stopping the movement of the second braking cone element or portion towards the first braking input element.

Additionally, the at least one hook may be a snap hook for forming a snap-in connection with the first braking cone element or portion. This allows assembling these two members, optionally with the first biasing or spring element arranged therebetween, as a simple, easily handleable subassembly and therefore further simplifies an assembly of the endoscope.

Preferably, at least one, preferably exactly three (or alternatively two, four, five or six) circumferential slits and/ or second cam structures and at least one, preferably exactly three (or alternatively two, four, five or six) first hooks including first cam structures are provided. This is a particularly stable and relatively simple configuration. In particular, the circumferential slits (e.g. one, two, three, four, five or six thereof) and the first hooks (e.g. one, two, three, four, five or six thereof) may extend along the same circumferential line.

It is considered expedient, if a free end portion of the at least one first hook forms the first cam structure and a surface of the first braking cone element or portion opposite to the first braking input element forms the second cam structure. Thus, the at least one hook may fulfil both the function of pulling the first and the second braking cone elements or portions apart when the first braking mechanism is opened and the function of controlling a process of opening and closing the first braking mechanism via the first cam pairing (i.e. via the first cam structure and the second cam structure).

Preferably, the first braking cone element or portion and/ or the second braking cone element or portion is elastically deformable at least in a radial direction. Alternatively or additionally, it may also be deformable in the axial direction, in particular less than in the radial direction. Expressed in other words, a radial position of portions, preferably each circumferential portion, of the first and/ or second braking cone element or portion is variable. I.e. the first and/ or second braking cone element or portion preferably forms or comprises one or more spring portions adapted for extending radially. This is advantageous since small misalignments of the first and second braking cone elements or portions with respect to each other can thus be compensated. Thus, a maximum amount of contact between the first and second braking surfaces can be ensured and a braking force generated by the first braking mechanism may be reproducibly achieved even if such alignment errors occur during assembly or due to manufacturing tolerances.

In particular, the first braking cone element or portion and/ or the second braking cone element or portion may advantageously form axially extending slits, particularly slits extending from one rim of the first and/ or second braking surface to provide elastically deformability. This is a particularly simple and cost efficient way to provide an elastically deformable first and/ or second braking cone element or portion. Alternatively, a material of the first and/ or second braking cones may be elastic or coated with a flexible material or corrugated or the like.

Advantageously, one among the first braking surface and the second braking surface comprises or consists of an amorphous plastic material, preferably ABS, and/ or another one among the first braking surface and the second braking surface comprises or consists of semi-crystalline plastic material, preferably PBT or PB or PP. This allows to provide an appropriate friction force between the first and second braking surfaces which may not be achieved with a material pairing consisting only of semi-crystalline or crystalline materials. Additionally, sticking of two surfaces formed by amorphous plastic material, particularly during increased temperatures, may be avoided. Further, if both braking surfaces were formed of the same material, a squeaking noise would occur when said braking surfaces slide against each other. Such a noise may be annoying to a user or leave a low quality impression. With the above-defined material pairing, generation of such a noise can be reduced or avoided. Preferably, the second braking surface may be formed from ABS and the first braking surface may be formed from PP.

In particular, it is preferred that an angle of the essentially cone-shaped first braking surface and/ or the essentially cone-shaped second braking surface with respect to the first input shaft is between 8 and 30 degrees, preferably between 10 and 20 degrees, further preferably between 14 and 16 degrees, further preferably 15 degrees. This corresponds to an optimum angle taking into consideration the following effects. The angle should be as low as possible so that an average distance of the first and second braking surfaces with respect to the axis is maximized. Said distance represents a lever for applying a braking torque generated by the braking contact between the first and second braking surfaces. I.e. said lever and thus, the braking torque is maximized. Also, in a formula for calculating the cone brake, a sinus of this angle is used as a denominator or divisor. Accordingly, if the angle is small, a value of said denominator is small and the braking torque is high. A minimum of the angle of the first and/or second braking surfaces is determined such that self-locking of the first and second braking surfaces due to a wedge effect is optimized or essentially prevented.

Preferably, the second braking cone element or portion may be formed integrally with the first steering input element. This reduces a number of members which have to be manufactured and assembled and thus costs. Alternatively, the second braking cone element or portion may be provided as a separate part or member arranged between the first steering input element and the first braking cone element. In this case, the first biasing or spring element may be arranged between the second braking cone element or portion and the first steering input element as described above. Also, a material for the second braking surface may be chosen more flexibly.

Moreover, it may be advantageous if the first braking cone element or portion and/ or the second braking cone element or portion include a carrier element and a friction cone stacked onto each other. The friction cone may form the first or second braking surface. The friction cone may be loosely arranged between the first or second braking cone element or portion and the carrier portion or may be arranged to be non-rotatable with respect to either one of them, e.g. via a spline geometry. The carrier element may provide a stiff basis for carrying the friction cone and provide stability. Thus, a robust carrier element may be chosen which may e.g. be suitable for supporting the first biasing or spring element as discussed above. Further, a material for the friction cone may be chosen more flexibly.

Preferably, the steering mechanism further comprises a manually operable second steering input element being provided for receiving a rotational steering input by the user and being connected to a second input shaft rotatably supported at the handle or interface housing, and a second braking mechanism for braking the second input shaft with respect to the handle or interface housing, the second braking mechanism comprising a manually operable second braking input element being provided for receiving a braking input by the user, and a third braking cone element or portion which forms an essentially cone-shaped third braking surface and is non-rotatably connected to the handle or interface housing, and a fourth braking cone element or portion which forms an essentially cone-shaped fourth braking surface and is non-rotatably connected to the second steering input element, with the third braking cone element or portion and/ or the fourth braking cone element or portion being axially shiftable via the second braking input element to bring the third braking surface into a braking contact with the fourth braking surface.

A second part of the steering mechanism including the manually operable second steering input element and the second braking mechanism may essentially be formed similarly to the above described first part of the steering mechanism including the manually operable first steering input element and the first braking mechanism in any of the above described basic or preferred configurations.

Preferably, the members forming the first and second parts of the steering mechanism are arranged in opposite order along the central axis. In particular, the second braking input element may be arranged axially adjacent to the handle or interface housing, the second steering input element may be arranged axially outwards adjacent to the second braking input element with the third and fourth braking cone elements or portions arranged therebetween, the first steering input element may be arranged axially outwards adjacent to the first steering input element and the first steering input element may be arranged axially outwards adjacent to the first braking input element with the first and second braking cone elements or portions arranged therebetween.

In the second part of the steering mechanism, the third braking cone element or portion may form at least one, preferably exactly three, exactly four or exactly five (or alternatively two or six) circumferential slits and/ or fourth cam structures and the second braking input element may correspondingly form at least one, preferably exactly three, exactly four or exactly five (or alternatively two or six) second hooks including third cam structures.

Further, instead of extending through circumferential slits in the third braking cone element or portion, the second hooks could extend through a central through hole, i.e. be located radially inside of the third braking cone element or portion engaging the third braking cone element or portion at the inner rim or diameter thereof, which could be a particularly simple and robust design. In this case, the third braking cone would not require circumferential slits. In other words, The second hooks may also be located on an inner portion of the third braking cone element or portion, so they do not necessarily penetrate it through cut-outs or circumferential slits formed therein but may also grab the third braking cone element or portion on an inner diameter portion thereof.

Further, the object underlying the present application is solved by a system comprising an above described endoscope and a monitor connectable to the endoscope.

### Brief description of figures

The following figures illustrate exemplary embodiments of the disclosure. The disclosure is not limited to the embodiments described below. Other embodiments, combinations of embodiments and modifications may be provided within the scope of protection defined by the claims.
Fig. 1 shows a schematic view of a system comprising an endoscope and a monitor according to the present disclosure.
Fig. 2 shows a cross-sectional view of a steering mechanism according to a first embodiment of the endoscope according to Fig. 1.
Fig. 3 shows a second braking cone element and a first braking input element of the steering mechanism of Fig. 2.
Fig. 4 shows a steering input element of the steering mechanism of Fig. 2.
Fig. 5 shows a second braking input element and a fourth braking cone element of the steering mechanism of Fig. 2.
Fig. 6 is a partial view of the steering mechanism of Fig. 2 illustrating an operation of said steering mechanism.
Fig. 7 shows a cross-sectional view of a steering mechanism according to a second embodiment of the endoscope according to Fig. 1.
Fig. 8 shows a perspective view of the first part of the steering mechanism of the first and/ or second embodiment.
Fig. 9 shows the first part of the steering mechanism of Fig. 8 with a first braking input element disassembled.

### Detailed description of preferred embodiments

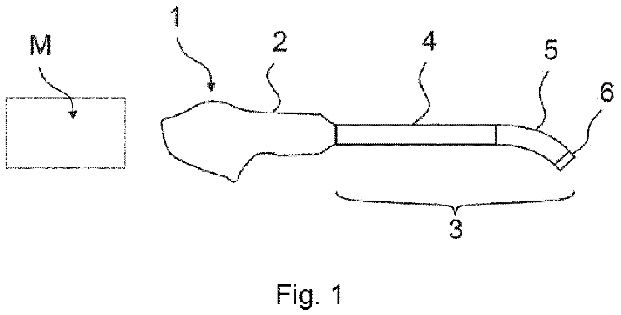

Fig. 1 shows a schematic view of an endoscope 1, which is preferably a single-use endoscope. The endoscope 1 has a proximal endoscope handle 2 and an insertion cord 3 extending distally from the endoscope handle 2. The insertion cord 3 has an insertion tube 4 connected to the endoscope handle 2. The insertion cord 3 further includes a bending section 5 connected to a distal end of the insertion tube 4 and a distal tip unit 6 connected to a distal end of the bending section 5.

At the distal tip unit 6, image capturing means such as a miniature video camera and illuminating means such as light-emitting diodes or optical fibers connected to a proximal source of light are arranged/installed, such that the patient's body cavity can be illuminated and inspected. An image captured by the image capturing means can be shown on a monitor M. The monitor M is provided separately from and connectable with the endoscope 1.

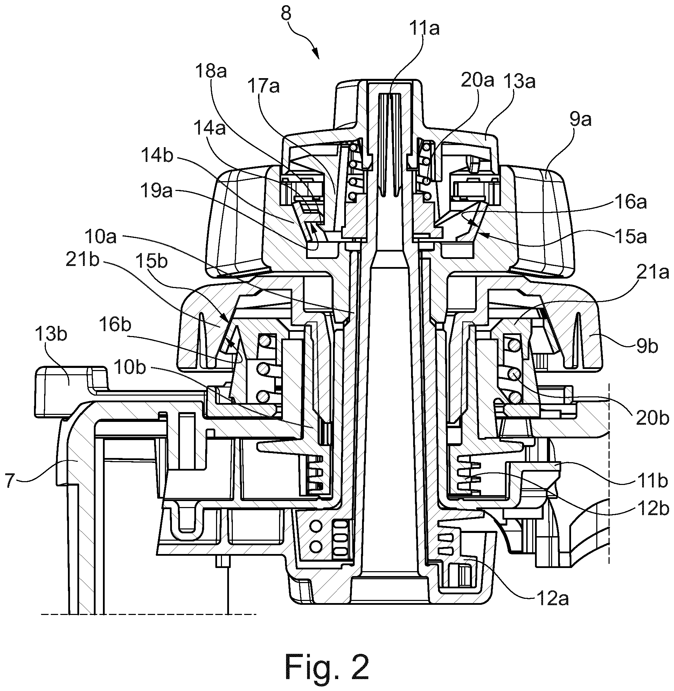

The bending section 5 is configured to perform a bending/ pivoting/ swivelling movement in four different directions/ in two bending planes, preferably with orthogonal angles between circumferentially adjacent directions. This enables a steering of the endoscope 1. The bending/ pivoting/ swivelling movement is controlled by a steering mechanism 8 which is not shown in Fig. 1 but described in detail below with reference to Figs. 2 to 7. In a neutral state, the bending section 5 is unbent by the steering mechanism 8, preferably straight/ in alignment with the insertion tube 4.

Fig. 2 shows a cross-sectional view of a portion of a handle housing 7 of the endoscope handle 2 where a steering mechanism 8 according to a first embodiment of the endoscope according to Fig. 1 is arranged. The steering mechanism 8 comprises a first portion for steering the distal tip unit 6 in a first pending plane and a second portion for steering the distal tip unit 6 in a second bending plane.

The first portion of the steering mechanism 8 comprises a first steering input element 9a, in particular a manipulator wheel. The first steering input element 9a is arranged at an outer side of the handle housing 7 and is fixedly connected to a first input shaft 10a.

The first input shaft 10a and the first steering input element 9a are rotatably supported on a first supporting rod 11a, which is a central rod connected fixedly to the handle housing 7. Expressed in other words, the handle housing 7 and the first supporting rod 11a are static parts and the first steering input element 9a and the first input shaft 10a are fixed together and can rotate around the first supporting rod 11a. The first input shaft 10a extends to an inside of the handle housing 7 where it forms or is connected to a first control wheel 12a. In particular, the first input shaft 10a and the first control wheel 12a are integrally formed as a single part, i.e. they form one and the same part, i.e. are formed material-integrally with each other. In other words, the first control wheel 12a may be an end stop segment of the first input shaft 10a. For example, the first control wheel 12a is a first wire drum for controlling a bending (e.g. left/ right bending) of the bending section 5. The first control wheel 12a (e.g. the first wire drum) is connected or connectable to a first pair of steering wires not illustrated in this figure. Further, a first braking input element 13a in the shape of a knob is snapped onto an outer end of the first supporting rod 11a so as to be rotatable and to be axially fixed.

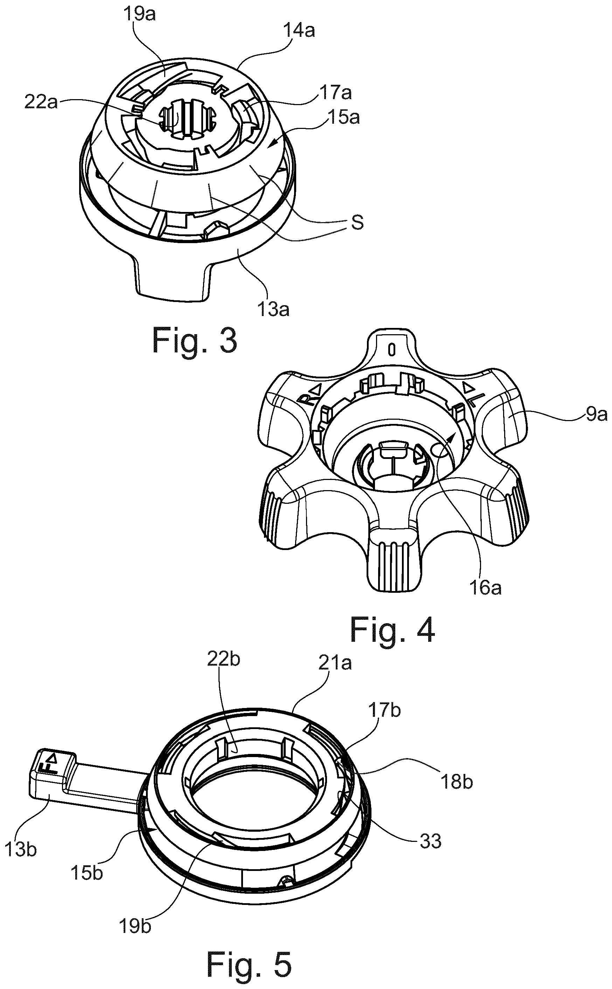

The first steering input element 9a and the first supporting rod 11a are selectively connectable via a first braking mechanism in order to brake a rotation of the first steering input element 9a and the first input shaft 10a. In particular, the first braking mechanism comprises a first braking cone element or portion 14a which is a ring-shaped part being non-rotatably and axially translatably connected to the first supporting rod 11a, e.g. via a spline geometry as shown in Fig. 3. The first braking cone element or portion 14a is essentially cone-shaped. Thus, at its radially outer surface, the first braking cone element or portion 14a forms a cone shaped first braking surface 15a, which, in the cross-sectional view, extends diagonally with respect to a central axis of the first supporting rod 11a.

Further, the first steering input element 9a forms a second braking cone element or portion 14b at its radially inner side. Said second braking cone element or portion 14b forms a radially inner surface which is essentially cone shaped and provides a second braking surface 16a. The second braking surface 16a has essentially the same angle with respect to the central axis as the first braking surface 15a. The first braking surface 15a faces radially outwards and towards the handle housing 7. The first braking surface 15a and the second braking surface 16a are arranged at positions opposite to each other and are adapted to come into braking contact with each other.

Said braking contact is achieved by moving the first braking cone element or portion 14a in an axial direction defined by the first supporting rod 11a. The axial movement of the first braking cone element or portion 14a is operated via the first braking input element 13a. In particular, the first braking input element 13a forms first hooks 17a, which are circumferentially spaced with respect to each other and extend axially so as to penetrate through circumferential slits formed in the first braking cone element or portion 14a. At their free ends, the first hooks 17a form a first cam structure 18a. The first cam structure 18a contacts a surface of the first braking cone element or portion 14a facing in the axial direction. Said surface serves as a second cam structure 19a, which is described in detail below with reference to Fig. 3.

Thus, via a first cam pairing formed by the first cam structure 18a and the second cam structure 19a, the first braking input element 13a is able to pull on the first braking cone element or portion 14a in order to translate it axially outwards. Further, radially inwards with regard to the first hooks 17a a first spring element 20a is arranged between the first braking input element 13a and the first braking cone element or portion 14a, biasing the first and second cam structures 18a, 19a to contact each other. Accordingly, by rotating the first braking input element 13a, the first cam structure 18a formed by the first hooks 17a glides circumferentially along the second cam structure 19a, pulling the first braking cone element or portion 14a away from the second braking cone element or portion 14b to open the first braking mechanism or letting the first spring element 20a push the first braking cone element or portion 14a against the second braking cone element or portion 14b in order to close the first braking mechanism. I.e. the first and second braking surfaces 15a, 16a can be brought into braking contact via the first spring element 20a and can be brought out of braking contact via the first cam pairing.

The first supporting rod 11a, the first input shaft 10a, the first steering input element 9a, the first braking input element 13a, and the first braking cone element or portion 14a are arranged coaxially with respect to each other.

The second portion of the steering mechanism 8 is arranged coaxially with the first portion of the steering mechanism 8, in particular radially outside of said first portion. The second portion of the steering mechanism 8 comprises a second steering input element 9b, in particular a manipulator wheel, which is preferably larger than the first steering input element 9a and further preferably is provided for effecting an up/ down bending of the bending section 5. The second steering input element 9b is arranged at an outer side of the handle housing 7 and is fixedly connected to a second input shaft 10b. The second input shaft 10b and the second steering input element 9b are rotatably supported on a second supporting rod 11b, which is a hollow rod accommodating the first supporting rod 11a and the first input shaft 9a and is connected fixedly to the handle housing 7. The second input shaft 10b extends to an inside of the handle housing 7 where it forms or is connected to a second control wheel 12b. Expressed in other words, the second input shaft 10b and the second control wheel 12b are two portions or one member, i.e. are formed material-integrally with each other. The second control wheel 12b (e.g. a second wire drum) is connected or connectable to second steering wires not illustrated in this figure. Further, a second braking input element 13b in the shape of a plate with a manipulator lever extending radially outwards is provided coaxially with the second input shaft 10b. The second braking input element 13b is rotatably and non-translatably supported at the handle housing 7.

The second steering input element 9b and the second supporting rod 11b are selectively connectable via a second braking mechanism in order to brake a rotation of the second steering input element 9b and the second input shaft 10b. In particular, the second braking mechanism comprises a third braking cone element or portion 21a. In particular, the third braking cone element or portion 21a is a ring-shaped part which is non-rotatably and axially translatably connected to the handle housing 7, e.g. via a spline geometry as shown in Fig. 5. The third braking cone element or portion 21a is essentially cone shaped. Thus, the third braking cone element or portion 21a provides, at its radially outer surface, a cone shaped third braking surface 15b, which, in the cross-sectional view, extends diagonally with respect to a central axis of the second supporting rod 11b.

Correspondingly, the second steering input element 9b forms on its radially inner side a fourth braking cone element or portion 21b. Said fourth braking cone element or portion 21b has a radially inner surface which is essentially cone shaped and provides a fourth braking surface 16b. The fourth braking surface 16b has essentially the same angle with respect to the central axis as the third braking surface 15b. The third braking surface faces radially outwards and away from the handle housing 7. The third braking surface 15b and the fourth braking surface 16b are arranged at a position opposite to each other and are adapted to come into braking contact with each other.

Said braking contact is achieved by moving the third braking cone element or portion 21a in the axial direction. The axial movement of the third braking cone element or portion 21a is operated via the second braking input element 13b. In particular, as illustrated in Fig. 5, the second braking input element 13b forms second hooks 17b, which are circumferentially spaced with respect to each other and extend axially so as to penetrate through circumferential slits formed in the third braking cone element or portion 21a. At their free ends, the second hooks 17b form a third cam structure 18b. The third cam structure 18b contacts a surface of the third braking cone element or portion 21a facing in the axial direction. Said surface serves as a fourth cam structure 19b. As an alternatively to the illustrated embodiment the hooks 17b could be located radially inside of the third braking cone element or portion 21a engaging the third braking cone element or portion 21a at the inner diameter thereof, which could be a particularly simple and robust design.

Thus, via a second cam pairing formed by the third cam structure 18b and the fourth cam structure19b, the second braking input element 13b is able to pull on the third braking cone element or portion 21a in order to translate it axially. Further, a second spring element 20b is arranged between the second braking input element 13b and the third braking cone element or portion 21a, biasing the third and fourth cam structures 18b, 19b to contact each other. Accordingly, by rotating the second braking input element 13b, the third cam structure 18b formed by the second hooks 17b glides circumferentially along the fourth cam structure 19b, pulling the third braking cone element or portion 21a away from the fourth braking cone element or portion 21b to open the second braking mechanism or letting the second spring element 20b push the third braking cone element or portion 21a against the fourth braking cone element or portion 21b in order to close the second braking mechanism.

The second supporting rod 11b, the second input shaft 10b, the second steering input element 9b, the second braking input element 13b, and the second braking cone element or portion 14b are arranged coaxially with respect to each other.

Fig. 3 shows the first braking cone element or portion 14a and the first braking input element 13a of the steering mechanism 8 of Fig. 2. In particular, the first braking cone element or portion 14a forms a first central through hole 22a for insertion of the first supporting rod 11a, with an inner surface of said first central through hole 22a forming the above discussed splined geometry. Further, the first braking cone element or portion 14a forms three circumferential slits through which the first hooks 17a reach to contact the second cam structure 19a. The second cam structure 19a includes three sections respectively extending adjacent to the circumferential slits. The second cam structure 19a forms a first flat end portion, a second flat end portion and an intermediate first ramp portion connecting the first and second flat end portions. The first flat end portion is formed by an axial end surface of the first braking cone element or portion 14a and the first ramp and the second flat end portion are recessed in said axial end surface. In Fig. 3, the first hooks 17a are in contact with the second flat end portion, such that the first braking cone element or portion 14a is pushed away from the first braking input element 13a and the first braking mechanism is closed.

Although three first hooks 17a with first cam structures 18a paired with three circumferential slits and/ or three second cam structures 19a of the first braking cone element or portion 14a are preferred, there may be exactly one, or two, four, five or six sets thereof. Similarly, although three second hooks 17b with third cam structures 18b paired with three circumferential slits and/ or three fourth cam structures 19b of the third braking cone element or portion 21a are preferred, there may be exactly one, or two, four, five or six sets thereof.

Further Fig. 3 shows slits S axially extending in the first braking surface 15a (optionally also in the second braking surface 16a) in order to provide elastic deformability. These may be provided in any of the described embodiments.

Fig. 4 shows a first steering input element 9a of the steering mechanism 8 of Fig. 2. The first steering input element 9a forms a receptacle for accommodating the first braking cone element or portion 14a and for supporting the first braking input element 13a. Further, a circumferential wall of said receptacle forms the second braking cone element or portion 14b, in particular the second braking cone surface 16a.

Fig. 5 shows a second braking input element 13b and a third braking cone element or portion 21a of the steering mechanism 8 of Fig. 2. Similarly to the first braking cone element or portion 14a, the third braking cone element or portion 21a forms three circumferential slits through which the second hooks 17b extend to contact the fourth cam structure 19b. The fourth cam structure 19b includes three sections respectively extending adjacent to the circumferential slits. The fourth cam structure 19b forms a third flat end portion, fourth flat end portion and an intermediate second ramp portion connecting the third and fourth flat end portions. The third flat end portion is formed by an axial end surface of the third braking cone element or portion 21a and the second ramp portion and the fourth flat end portion are recessed in said axial end surface. In Fig. 5, the second hooks 17b are in contact with the third flat end portion, such that the third braking cone element or portion 21a is pushed away from the second braking input element 13b and the second braking mechanism is closed.

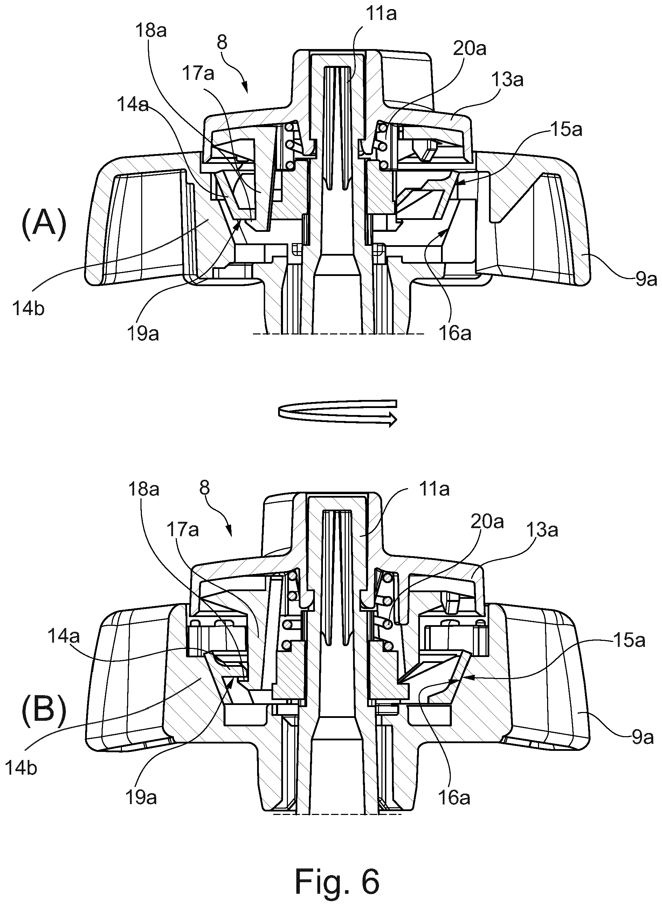

Fig. 6 is a partial view of the first part of the steering mechanism 8 of Fig. 2 illustrating an operation of said steering mechanism 8. In view (A), the first braking mechanism is open. I.e. the first braking input element 13a is rotated such that the first cam structure 18a formed by the first hooks 17a contacts the first flat end portions of the second cam structure 19a, thus pulling the first braking cone element or portion 14a including the first braking surface 15a away from the second braking cone element or portion 14b including the second braking surface 16a. By rotating the first braking input element 13a as illustrated via an arrow in Fig. 6, the first hooks 17a shift along the first ramp to the second flat end portion of the second cam structure 19a until the position described with respect to Fig. 3 is reached. In principle, the first braking input element 13a (i.e. the brake lever or knob) and the second cam structure 19a can be designed to rotate either clockwise or counter clockwise in order to close the braking mechanism. Thus, the first spring element 20a pushes the first braking cone element 14a towards the second braking cone element 14b and closes the first braking mechanism. This state is shown in Fig. 6, view (B) where the first and second braking surfaces 15a, 16a are in contact with each other.

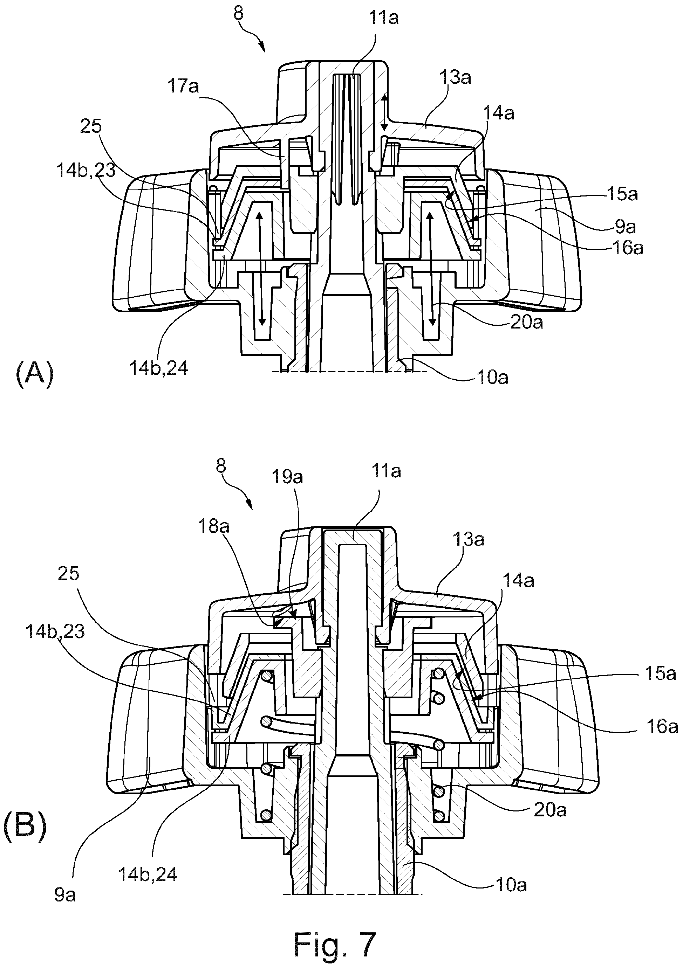

Fig. 7 shows a cross-sectional view of a first part of a steering mechanism 8 according to a second embodiment of the endoscope 1 according to Fig. 1. Similarly to the first embodiment, said second part of the steering mechanism 8 includes a first steering input element 9a, in particular a manipulator wheel. The first steering input element 9a is arranged similarly to the one in the first embodiment with respect to the handle housing 7, a first input shaft 10a, a first supporting rod 11a and a first control wheel 12a, e.g. a first wire drum. The latter is not shown in Fig. 7, but may be formed and connected to the first input shaft 10a in a similar manner as disclosed in Fig. 2. Further, a first braking input element 13a in the shape of a knob is snapped onto an outer end of the first supporting rod 11a so as to be rotatable and to be axially fixed.

The first steering input element 9a and the first supporting rod 11a are selectively connectable via a first braking mechanism in order to brake a rotation of the first steering input element 9a and the first input shaft 10a. In particular, the first braking mechanism comprises a first braking cone element or portion 14a which is a ring-shaped part being non-rotatably and axially translatably connected to the first supporting rod 11a, e.g. via a spline geometry. The first braking cone element or portion 14a is essentially cone-shaped. Thus, at its radially inner surface, the first braking cone element or portion 14a forms a cone shaped first braking surface 15a, which, in the cross-sectional view, extends diagonally with respect to a central axis of the first supporting rod 11a. The first braking surface faces radially inwards and towards the handle housing 7.

Further, a second braking cone element or portion 14b is arranged between the first braking input element 13a and the first steering input element 9a. The second braking cone element or portion 14b is axially translatably and non-rotatably supported at a radially inner side of the first steering input element 9a. Said second braking cone element or portion 14b has a friction cone 23 with a radially outer surface which is essentially cone shaped and provides a second braking surface 16a. The friction cone 23 is supported on a spring holder element 24. The spring holder element 24 forms a supporting surface facing towards the friction cone 23. Opposite thereto, the spring holder element 24 forms a receptacle for accommodating and supporting a first spring element 20a (shown only schematically via arrows). The first spring element 20a is arranged between the second friction cone element or portion 14b and the first steering input element 9a. The second braking surface 16a has essentially the same angle with respect to the central axis as the first braking surface 15a and a supporting surface of the spring holder element 24 supporting the friction cone 23. The first braking surface 15a and the second braking surface 16a are arranged at positions opposite to each other and are adapted to come into braking contact with each other.

Said braking contact is achieved by moving the first braking cone element or portion 14a in an axial direction defined by the first supporting rod 11a. The axial movement of the first braking cone element or portion 14a is operated via the first braking input element 13a. In particular, the first braking input element 13a forms a first cam structure 18a facing towards the first braking cone element 14a and contacting a second cam structure 19a formed thereon. The first and/ or the second cam structures 18a, 19a (i.e. the first cam pairing) form a ramp such that a rotation of the first braking input element 13a in one direction will result in the first cam pairing pushing the first braking cone element 14a against the spring towards the second braking cone element or portion 14b in order to close the first braking mechanism as shown in Fig. 7, view (A).

Additionally, the first braking input element 13a forms first hooks 17a, which are circumferentially spaced with respect to each other and extend axially so as to penetrate through circumferential slits formed in the first braking cone element or portion 14a. At their free ends, the first hooks 17a contact a surface of the first braking cone element or portion 14a facing in the axial direction to pull said first braking cone element or portion 14a away from the second braking cone element or portion 14b when the first braking mechanism is opened. The second braking cone element or portion 14b is held back via a stop portion 25 formed at the first steering input element 9a such that a gap is formed between the first and second braking cone portions or elements 14a, 14b when the first braking mechanism is opened as shown in Fig. 7, view (B).

Thus, via the first cam pairing formed by the first cam structure 18a and the second cam structure 19a, the first braking input element 13a is able to push the first braking cone element or portion 14a in order to translate it axially towards the second braking cone element or portion 14b. Accordingly, by rotating the first braking input element 13a, the first cam structure 18a glides circumferentially along the second cam structure 19a, pushing the first braking cone element or portion 14a against a biasing force of the first spring element 20a towards the second braking cone element or portion 14b to close the first braking mechanism or pulling the first braking cone element or portion 14a via the first hooks 17a away from the second braking cone element or portion 14b in order to open the first braking mechanism.

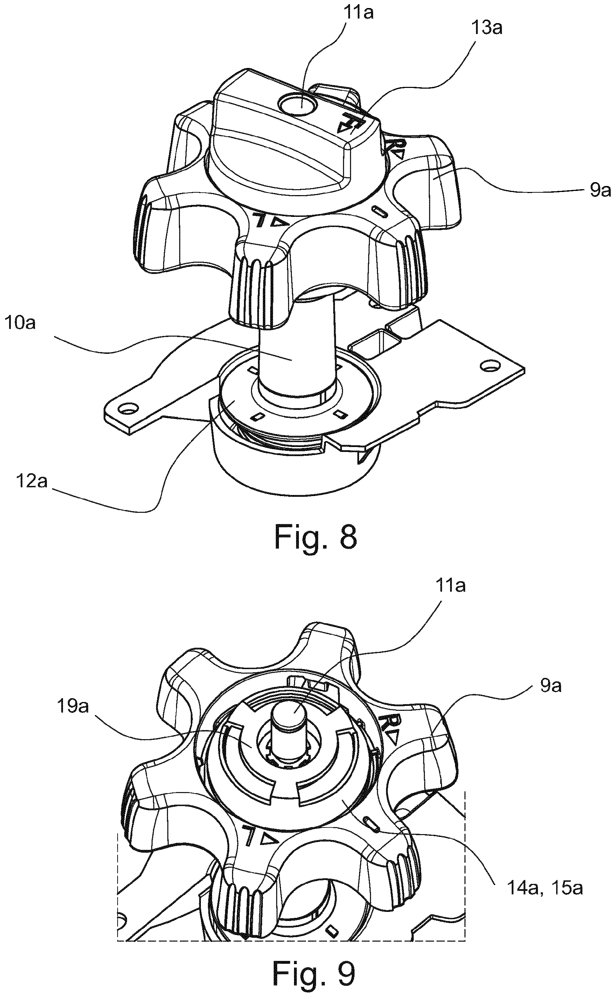

Fig. 8 shows a perspective view of the first part of the steering mechanism of the first and/ or second embodiment disclosed above. Fig. 9 shows the first part of the steering mechanism of Fig. 8 with a first braking input element disassembled. In the latter, the circumferential slits in the first braking cone element or portion 14a are shown. Further, an axial surface of the first braking cone element or portion 14a which forms or is adapted to carry the second cam structure 19a is shown.

The term cone should in the present context be understood to include the shape of a truncated cone. At present it is preferred that the cone is a true cone geometry with the outer surface forming a straight line from the smallest diameter at an axial end position to the largest diameter at the other axial end position, which will be the simplest version to design and produce. However "essential cone shaped" should be understood to include a shape with a cross-section, which is not perfectly circular, such as slightly wavy or provided with ribs or grooves, and further the cone surface may bulge, e.g. so that the outer surface does not form a straight line from an axial end position to the other end axial position, but instead a curved line, such as a circular section or a number of circular sections, a combination of straight lines with different gradient, or a combination of curved and straight lines. The combination of ribs and/or grooves on mating cones could provide an extra geometrical constraint to be overcome in the brake-on position, i.e. a higher amount of braking.

The angles of any two cone elements, i.e. the first and second or third and fourth, may be the same or the angles may differ slightly. This could further increase braking function in practice, as the mating cones could be designed to ensure contact at the largest diameter, also in case of slight misalignment or variance in production. Additionally disengagement of the brake may be facilitated.

The amount of braking can be tailored to the specific preferences by e.g. the choice of spring constant, material (friction coefficient) or angle of the cones (braking cone elements).

At present an amount of braking considered relevant is in the order of 25-250 Nmm, such as 50-200 Nmm. Examples could be in the range of 55-70 Nmm for the first steering input element (having the smallest diameter), and in the range of 160-200 Nmm for the second steering input element (having the largest diameter).

Preferably the amount of braking should not be too high, as this could pose a risk of injury if a user accidentally pulls out the endoscope in a bent configuration with brake activated. Instead, the endoscope should preferably straighten out by overcoming the brake. Further it may be an advantage if the user can fine-tune the amount of bending even with brake activated.

### List of reference numbers

- 1: Endoscope
- 2: Endoscope handle
- 3: Insertion cord
- 4: Insertion tube
- 5 9a,: Bending section
- 6: Distal tip unit
- 7: Handle housing
- 8: Steering mechanism
- 9b: First and second steering input elements
- 10a, 10b: First and second input shafts
- 11a, 11b: First and second supporting rods
- 12a, 12b: First and second control wheels
- 13a, 13b: First and second braking input elements
- 14a, 14b: First and second braking cone elements or portions
- 15a, 15b: First and third braking surfaces
- 16a, 16b: Second and fourth braking surfaces
- 17a, 17b: First and second hooks
- 18a, 18b: First and third cam structures
- 19a, 19b: Second and fourth cam structures
- 20a, 20b: First and second biasing or spring elements
- 21a, 21b: Third and fourth braking cone elements or portions
- 22a, 22b: First and second central through holes
- 23: Friction cone
- 24: Spring holder element
- 25: Stop portion
- M: Monitor
- S: Slits

## Claims

1. An endoscope (1) comprising:
a proximal endoscope handle or interface (2) having a handle or interface housing (7);
an insertion cord (3) extending from the endoscope handle or interface (2) and configured to be inserted into a patient's body cavity, the insertion cord (3) comprising an insertion tube (4), a bending section (5) and a distal tip unit (6); and
a steering mechanism (8) configured to swivel the distal tip unit (6) by bending the bending section (5) at least in a first bending plane, the steering mechanism (8) comprising
a manually operable first steering input element (9a) being provided for receiving a rotational steering input by a user and being rotatably supported at the handle or interface housing (7); and
a first braking mechanism for braking the first steering input element (9a) with respect to the handle or interface housing (7), the first braking mechanism comprising
a manually operable first braking input element (13a) being provided for receiving a braking input by a user, **characterised in that** the first braking mechanism further comprises
a first braking cone element or portion (14a) which forms an essentially cone-shaped first braking surface (15a) and is non-rotatably connected to the handle or interface housing (7), and a second braking cone element or portion (14b) which forms an essentially cone-shaped second braking surface (16a) and is non-rotatably connected to the first steering input element or portion (9a), with the first braking cone element or portion (14a) and/ or the second braking cone element or portion (14b) being axially translatable via the first braking input element (15) to press the first braking surface (15a) against the second braking surface (16a).

2. The endoscope according to claim 1, wherein the first braking input element (13a) forms a first cam structure (18a) and the first braking cone element or portion (14a) comprises a second cam structure (19a) which contacts the first cam surface (18a) such that a rotation of the first braking input element (13a) is transformed into an axial translation of the first braking cone element or portion (14a).

3. The endoscope according to claim 2, wherein a first biasing or spring element (20a) is provided, which biases the first cam structure (18a) towards the second cam structure (19a), the first biasing or spring element (20a) preferably being arranged between the first braking input element (13a) and the first braking cone element or portion (14a).

4. The endoscope according to claim 3, wherein the first biasing or spring element (20a) is arranged between the first braking input element (13a) and the first braking cone element or portion (14a) or between the second braking cone element or portion (14b) and the first steering input element (9a).

5. The endoscope according to claim 3 or 4, wherein the first biasing or spring element (20a) is supported radially inside the first braking cone element or portion (14a) at a side opposite to the first braking surface (15a) or radially inside the second braking cone element (14b) at a side opposite to the second braking surface (16a).

6. The endoscope according to one of the claims 1 to 5, wherein the first braking cone element or portion (9a) forms at least one circumferential slit and the first braking input element (13a) forms at least one first hook (17a) extending through the at least one circumferential slit.

7. The endoscope according to claims 2 and 6, wherein a free end portion of the at least one first hook (17a) forms the first cam structure (18a) and a surface of the first braking cone element or portion (14a) opposite to the first braking input element (14a) forms the second cam structure (19a).

8. The endoscope according to one of the claims 1 to 7, wherein the first braking cone element or portion (14a) and/ or the second braking cone element or portion (14b) is elastically deformable at least in a radial direction.

9. The endoscope according to claim 8, wherein the first braking cone element or portion (14a) and/ or the second braking cone element or portion (14b) forms axially extending slits (S), particularly slits (S) extending from one rim of the first braking surface (15a) and/ or the second braking surface (16a) to provide elastic deformability.

10. The endoscope according to one of the claims 1 to 9, wherein one among the first braking surface (15a) and the second braking surface (16a) comprises or consists of an amorphous plastic material, preferably ABS, and another one among the first braking surface (15a) and the second braking surface (16a) comprises or consists of semi-crystalline plastic material, preferably PBT or PB or PP.

11. The endoscope according to one of the claims 1 to 10, wherein an angle of the essentially cone-shaped first braking surface (15a) and/ or the essentially cone-shaped second braking surface (16a) with respect to a central axis is between 8 and 30 degrees, preferably between 10 and 20 degrees, further preferably between 14 and 16 degrees, further preferably 15 degrees.

12. The endoscope according to one of the claims 1 to 11, wherein the second braking cone element or portion (14b) is formed integrally with the first steering input element (9a) or is provided as a separate part arranged between the first steering input element (9a) and the first braking cone element or portion (14a).

13. The endoscope according to one of the claims 1 to 12, wherein the first braking cone element or portion (14a) and/ or the second braking cone element or portion (14b) includes a carrier element and a friction cone (23) stacked onto each other.

14. The endoscope according to any one of the preceding claims 1 to 13, wherein the steering mechanism comprises:
a manually operable second steering input element (9b) being provided for receiving a rotational steering input by the user and being connected to a second input shaft (10b) rotatably supported at the handle or interface housing (7); and
a second braking mechanism for braking the second input shaft (10b) with respect to the handle or interface housing (7), the second braking mechanism comprising
a manually operable second braking input element (13b) being provided for receiving a braking input by the user, and
a third braking cone element or portion (21a) which forms an essentially cone-shaped third braking surface (15b) and is non-rotatably connected to the handle or interface housing (7), and a fourth braking cone element or portion (21b) which forms an essentially cone-shaped fourth braking surface (16b) and is non-rotatably connected to the second steering input element (9b), with the third braking cone element or portion (21a) and/ or the fourth braking cone element or portion (21b) being axially shiftable via the second braking input element (13b) to bring the third braking surface (15b) into a braking contact with the fourth braking surface (16b).

15. A system comprising an endoscope (1) according to any one of the claims 1 to 14 and a monitor (M) connectable to the endoscope (1).

## Patentansprüche

1. Endoskop (1), mit:
einem proximalen Endoskopgriff oder -schnittstelle (2) mit einem Griff- oder Schnittstellengehäuse (7);
einem Einführstrang (3), der sich von dem Endoskopgriff oder -schnittstelle (2) erstreckt und dazu ausgebildet ist, in eine Körperhöhle eines Patienten eingeführt zu werden, wobei der Einführstrang (3) ein Einführrohr (4), einen Biegeabschnitt (5) und eine distale Spitzeneinheit (6) aufweist; und
einem Lenkmechanismus (8), der dazu ausgebildet ist, die distale Spitzeneinheit (6) durch Biegen des Biegeabschnitts (5) zumindest in einer ersten Biegeebene zu schwenken, wobei der Lenkmechanismus (8) Folgendes aufweist:
ein manuell betätigbares erstes Lenkeingabeelement (9a), das zum Empfangen einer Drehlenkeingabe durch einen Benutzer vorgesehen ist und drehbar an dem Griff- oder Schnittstellengehäuse (7) gelagert ist; und
einen ersten Bremsmechanismus zum Bremsen des ersten Lenkeingabeelements (9a) in Bezug auf das Griff- oder Schnittstellengehäuse (7), wobei der erste Bremsmechanismus Folgendes aufweist:
ein manuell betätigbares erstes Bremseingabeelement (13a), das zum Empfangen einer Bremseingabe durch einen Benutzer vorgesehen ist, **dadurch gekennzeichnet, dass** der erste Bremsmechanismus ferner Folgendes aufweist:
ein erstes Bremskegelelement oder -abschnitt (14a), das eine im Wesentlichen kegelförmige erste Bremsfläche (15a) bildet und drehfest mit dem Griff- oder Schnittstellengehäuse (7) verbunden ist, und ein zweites Bremskegelelement oder - abschnitt (14b), das eine im Wesentlichen kegelförmige zweite Bremsfläche (16a) bildet und drehfest mit dem ersten Lenkeingabeelement oder -abschnitt (9a) verbunden ist, wobei das erste Bremskegelelement oder -abschnitt (14a) und/oder das zweite Bremskegelelement oder -abschnitt (14b) über das erste Bremseingabeelement (15) axial verschiebbar ist, um die erste Bremsfläche (15a) gegen die zweite Bremsfläche (16a) zu drücken.

2. Endoskop nach Anspruch 1, wobei das erste Bremseingabeelement (13a) eine erste Nockenstruktur (18a) bildet und das erste Bremskegelelement oder -abschnitt (14a) eine zweite Nockenstruktur (19a) aufweist, die die erste Nockenfläche (18a) berührt, so dass eine Drehung des ersten Bremseingabeelements (13a) in eine axiale Verschiebung des ersten Bremskegelelements oder -abschnitts (14a) umgewandelt wird.

3. Endoskop nach Anspruch 2, wobei ein erstes Vorspann- oder Federelement (20a) vorgesehen ist, das die erste Nockenstruktur (18a) in Richtung der zweiten Nockenstruktur (19a) vorspannt, wobei das erste Vorspann- oder Federelement (20a) vorzugsweise zwischen dem ersten Bremseingabeelement (13a) und dem ersten Bremskegelelement oder -abschnitt (14a) angeordnet ist.

4. Endoskop nach Anspruch 3, wobei das erste Vorspann- oder Federelement (20a) zwischen dem ersten Bremseingabeelement (13a) und dem ersten Bremskegelelement oder -abschnitt (14a) oder zwischen dem zweiten Bremskegelelement oder -abschnitt (14b) und dem ersten Lenkeingabeelement (9a) angeordnet ist.

5. Endoskop nach Anspruch 3 oder 4, wobei das erste Vorspann- oder Federelement (20a) radial innerhalb des ersten Bremskegelelements oder -abschnitts (14a) an einer der ersten Bremsfläche (15a) gegenüberliegenden Seite oder radial innerhalb des zweiten Bremskegelelements (14b) an einer der zweiten Bremsfläche (16a) gegenüberliegenden Seite gelagert ist.

6. Endoskop nach einem der Ansprüche 1 bis 5, wobei das erste Bremskegelelement oder -abschnitt (9a) mindestens einen Umfangsschlitz bildet und das erste Bremseingabeelement (13a) mindestens einen ersten Haken (17a) bildet, der sich durch den mindestens einen Umfangsschlitz erstreckt.

7. Endoskop nach den Ansprüchen 2 und 6, wobei ein freier Endabschnitt des mindestens einen ersten Hakens (17a) die erste Nockenstruktur (18a) bildet und eine dem ersten Bremseingabeelement (14a) gegenüberliegende Fläche des ersten Bremskegelelements oder -abschnitts (14a) die zweite Nockenstruktur (19a) bildet.

8. Endoskop nach einem der Ansprüche 1 bis 7, wobei das erste Bremskegelelement oder -abschnitt (14a) und/oder das zweite Bremskegelelement oder - abschnitt (14b) mindestens in einer radialen Richtung elastisch verformbar ist.

9. Endoskop nach Anspruch 8, wobei das erste Bremskegelelement oder - abschnitt (14a) und/oder das zweite Bremskegelelement oder -abschnitt (14b) sich axial erstreckende Schlitze (S) bildet, insbesondere Schlitze (S), die sich von einem Rand der ersten Bremsfläche (15a) und/oder der zweiten Bremsfläche (16a) erstrecken, um eine elastische Verformbarkeit bereitzustellen.

10. Endoskop nach einem der Ansprüche 1 bis 9, wobei entweder die erste Bremsfläche (15a) oder die zweite Bremsfläche (16a) ein amorphes Kunststoffmaterial, vorzugsweise ABS, umfasst oder daraus besteht, und die jeweils andere der ersten Bremsfläche (15a) und der zweiten Bremsfläche (16a) ein halbkristallines Kunststoffmaterial, vorzugsweise PBT oder PB oder PP, umfasst oder daraus besteht.

11. Endoskop nach einem der Ansprüche 1 bis 10, wobei ein Winkel der im Wesentlichen kegelförmigen ersten Bremsfläche (15a) und/oder der im Wesentlichen kegelförmigen zweiten Bremsfläche (16a) in Bezug auf eine Mittelachse zwischen 8 und 30 Grad, vorzugsweise zwischen 10 und 20 Grad, weiter vorzugsweise zwischen 14 und 16 Grad, weiter vorzugsweise 15 Grad beträgt.

12. Endoskop nach einem der Ansprüche 1 bis 11, wobei das zweite Bremskegelelement oder -abschnitt (14b) einstückig mit dem ersten Lenkeingabeelement (9a) ausgebildet ist oder als separates Teil vorgesehen ist, das zwischen dem ersten Lenkeingabeelement (9a) und dem ersten Bremskegelelement oder -abschnitt (14a) angeordnet ist.

13. Endoskop nach einem der Ansprüche 1 bis 12, wobei das erste Bremskegelelement oder -abschnitt (14a) und/oder das zweite Bremskegelelement oder - abschnitt (14b) ein Trägerelement und einen Reibungskegel (23) umfasst, die aufeinander gestapelt sind.

14. Endoskop nach einem der vorhergehenden Ansprüche 1 bis 13, wobei der Lenkmechanismus Folgendes aufweist:
ein manuell betätigbares zweites Lenkeingabeelement (9b), das zum Empfangen einer Drehlenkeingabe durch den Benutzer vorgesehen ist und mit einer zweiten Eingabewelle (10b) verbunden ist, die drehbar an dem Griff- oder Schnittstellengehäuse (7) gelagert ist; und
einen zweiten Bremsmechanismus zum Bremsen der zweiten Eingabewelle (10b) in Bezug auf das Griff- oder Schnittstellengehäuse (7), wobei der zweite Bremsmechanismus Folgendes aufweist:
ein manuell betätigbares zweites Bremseingabeelement (13b), das zum Empfangen einer Bremseingabe durch den Benutzer vorgesehen ist, und
ein drittes Bremskegelelement oder -abschnitt (21a), das eine im Wesentlichen kegelförmige dritte Bremsfläche (15b) bildet und drehfest mit dem Griff- oder Schnittstellengehäuse (7) verbunden ist, und ein viertes Bremskegelelement oder - abschnitt (21b), das eine im Wesentlichen kegelförmige vierte Bremsfläche (16b) bildet und drehfest mit dem zweiten Lenkeingabeelement (9b) verbunden ist, wobei das dritte Bremskegelelement oder -abschnitt (21a) und/oder das vierte Bremskegelelement oder - abschnitt (21b) über das zweite Bremseingabeelement (13b) axial verschiebbar ist, um die dritte Bremsfläche (15b) in einen Bremskontakt mit der vierten Bremsfläche (16b) zu bringen.

15. System mit einem Endoskop (1) nach einem der Ansprüche 1 bis 14 und einem Monitor (M), der mit dem Endoskop (1) verbindbar ist.

## Revendications

1. Endoscope (1) comprenant :
une poignée ou une interface d'endoscope proximale (2) présentant un boîtier de poignée ou d'interface (7) ;
un cordon d'insertion (3) s'étendant depuis la poignée, ou l'interface, d'endoscope (2) et configuré pour être inséré dans une cavité corporelle d'un patient, le cordon d'insertion (3) comprenant un tube d'insertion (4), une section de flexion (5) et une unité de pointe distale (6) ; et
un mécanisme de direction (8) configuré pour faire pivoter l'unité de pointe distale (6) en fléchissant la section de flexion (5) au moins dans un premier plan de flexion, le mécanisme de direction (8) comprenant
un premier élément d'entrée de direction pouvant être actionné manuellement (9a) qui est prévu pour recevoir une entrée de direction rotative par un utilisateur et qui est supporté de manière rotative au niveau du boîtier de poignée ou d'interface (7) ; et
un premier mécanisme de freinage pour freiner le premier élément d'entrée de direction (9a) par rapport au boîtier de poignée ou d'interface (7), le premier mécanisme de freinage comprenant
un premier élément d'entrée de freinage pouvant être actionné manuellement (13a) qui est prévu pour recevoir une entrée de freinage par un utilisateur, **caractérisé en ce que** le premier mécanisme de freinage comprend en outre
un premier élément, ou une première partie, de cône de freinage (14a) qui forme une première surface de freinage essentiellement en forme de cône (15a) et qui est relié(e) de manière non rotative au boîtier de poignée ou d'interface (7), et un deuxième élément, ou une deuxième partie, de cône de freinage (14b) qui forme une deuxième surface de freinage essentiellement en forme de cône (16a) et qui est relié(e) de manière non rotative au premier élément, ou à la première partie, d'entrée de direction (9a), le premier élément, ou la première partie, de cône de freinage (14a) et/ou le deuxième élément, ou la deuxième partie, de cône de freinage (14b) pouvant être déplacé(e)s axialement en translation par le biais du premier élément d'entrée de freinage (15) pour presser la première surface de freinage (15a) contre la deuxième surface de freinage (16a).

2. Endoscope selon la revendication 1, dans lequel le premier élément d'entrée de freinage (13a) forme une première structure de came (18a) et le premier élément, ou la première partie, de cône de freinage (14a) comprend une seconde structure de came (19a) qui vient en contact avec la première surface de came (18a) de telle sorte qu'une rotation du premier élément d'entrée de freinage (13a) soit transformée en une translation axiale du premier élément, ou de la première partie, de cône de freinage (14a).

3. Endoscope selon la revendication 2, dans lequel un premier élément de sollicitation ou de ressort (20a) est prévu, qui sollicite la première structure de came (18a) vers la seconde structure de came (19a), le premier élément de sollicitation ou de ressort (20a) étant, de préférence, agencé entre le premier élément d'entrée de freinage (13a) et le premier élément, ou la première partie, de cône de freinage (14a).

4. Endoscope selon la revendication 3, dans lequel le premier élément de sollicitation ou de ressort (20a) est agencé entre le premier élément d'entrée de freinage (13a) et le premier élément, ou la première partie de cône, de freinage (14a) ou entre le deuxième élément, ou la deuxième partie, de cône de freinage (14b) et le premier élément d'entrée de direction (9a).

5. Endoscope selon la revendication 3 ou 4, dans lequel le premier élément de sollicitation ou de ressort (20a) est supporté radialement à l'intérieur du premier élément, ou de la première partie, de cône de freinage (14a) sur un côté opposé à la première surface de freinage (15a) ou radialement à l'intérieur du deuxième élément de cône de freinage (14b) sur un côté opposé à la deuxième surface de freinage (16a).

6. Endoscope selon l'une des revendications 1 à 5, dans lequel le premier élément, ou la première partie, de cône de freinage (9a) forme au moins une fente circonférentielle et le premier élément d'entrée de freinage (13a) forme au moins un premier crochet (17a) s'étendant à travers la au moins une fente circonférentielle.

7. Endoscope selon les revendications 2 et 6, dans lequel une partie d'extrémité libre du au moins un premier crochet (17a) forme la première structure de came (18a) et une surface du premier élément, ou de la première partie, de cône de freinage (14a) opposée au premier élément d'entrée de freinage (14a) forme la seconde structure de came (19a).

8. Endoscope selon l'une des revendications 1 à 7, dans lequel le premier élément, ou la première partie, de cône de freinage (14a) et/ou le deuxième élément, ou la deuxième partie, de cône de freinage (14b) sont déformables élastiquement au moins dans une direction radiale.

9. Endoscope selon la revendication 8, dans lequel le premier élément, ou la première partie, de cône de freinage (14a) et/ou le deuxième élément, ou la deuxième partie, de cône de freinage (14b) forment des fentes s'étendant axialement (S), en particulier des fentes (S) s'étendant depuis un rebord de la première surface de freinage (15a) et/ou de la deuxième surface de freinage (16a) pour fournir une déformabilité élastique.

10. Endoscope selon l'une des revendications 1 à 9, dans lequel une première parmi la première surface de freinage (15a) et la deuxième surface de freinage (16a) comprend, ou est composée d'un matériau plastique amorphe, de préférence de l'ABS, et l'autre parmi la première surface de freinage (15a) et la deuxième surface de freinage (16a) comprend ou est composée d'un matériau plastique semi-cristallin, de préférence PBT ou PB ou PP.

11. Endoscope selon l'une des revendications 1 à 10, dans lequel un angle de la première surface de freinage essentiellement conique (15a) et/ou de la deuxième surface de freinage essentiellement conique (16a) par rapport à un axe central est entre 8 et 30 degrés, de préférence entre 10 et 20 degrés, de préférence encore entre 14 et 16 degrés, de préférence encore 15 degrés.

12. Endoscope selon l'une des revendications 1 à 11, dans lequel le deuxième élément, ou la deuxième partie, de cône de freinage (14b) est formé(e) d'un seul tenant avec le premier élément d'entrée de direction (9a) ou est prévu(e) sous la forme d'une pièce séparée agencée entre le premier élément d'entrée de direction (9a) et le premier élément, ou la première partie, de cône de freinage (14a).

13. Endoscope selon l'une des revendications 1 à 12, dans lequel le premier élément, ou la première partie, de cône de freinage (14a) et/ou le deuxième élément, ou la deuxième partie, de cône de freinage (14b) incluent un élément porteur et un cône de friction (23) empilés l'un sur l'autre.

14. Endoscope selon l'une quelconque des revendications précédentes 1 à 13, dans lequel le mécanisme de direction comprend :
un second élément d'entrée de direction pouvant être actionné manuellement (9b), prévu pour recevoir une entrée de direction rotative par l'utilisateur et étant relié à un second arbre d'entrée (10b) supporté de manière rotative au niveau du boîtier de poignée ou d'interface (7) ; et
un second mécanisme de freinage pour freiner le second arbre d'entrée (10b) par rapport au boîtier de poignée ou d'interface (7), le second mécanisme de freinage comprenant
un second élément d'entrée de freinage pouvant être actionné manuellement (13b) et prévu pour recevoir une entrée de freinage par l'utilisateur, et
un troisième élément, ou une troisième partie, de cône de freinage (21a) qui forme une troisième surface de freinage essentiellement en forme de cône (15b) et qui est relié(e) de manière non rotative au boîtier de poignée ou d'interface (7), et un quatrième élément, ou une quatrième partie, de cône de freinage (21b) qui forme une quatrième surface de freinage essentiellement en forme de cône (16b) et qui est relié(e) de manière non rotative au second élément d'entrée de direction (9b), le troisième élément, ou la troisième partie, de cône de freinage (21a) et/ou le quatrième élément, ou la quatrième partie, de cône de freinage (21 b) pouvant être déplacé(e)s axialement par le biais du second élément d'entrée de freinage (13b) pour amener la troisième surface de freinage (15b) en contact de freinage avec la quatrième surface de freinage (16b).

15. Système comprenant un endoscope (1) selon l'une quelconque des revendications 1 à 14 et un moniteur (M) pouvant être relié à l'endoscope (1).
